(19) Europäisches Patentamt
European Patent Office
Office européen des brevets

(11) **EP 3 193 962 B1**

(12) **EUROPÄISCHE PATENTSCHRIFT**

(45) Veröffentlichungstag und Bekanntmachung des Hinweises auf die Patenterteilung:
**13.07.2022 Patentblatt 2022/28**

(21) Anmeldenummer: **15766750.2**

(22) Anmeldetag: **15.09.2015**

(51) Internationale Patentklassifikation (IPC):
**A61M 1/14** (2006.01)     **A61M 1/34** (2006.01)
**A61B 5/00** (2006.01)

(52) Gemeinsame Patentklassifikation (CPC):
**A61M 1/1613; A61B 5/4836; A61B 5/4848; A61B 5/4875; A61M 1/14; A61M 1/34; A61M 1/341;** A61B 5/0537

(86) Internationale Anmeldenummer:
**PCT/EP2015/001846**

(87) Internationale Veröffentlichungsnummer:
**WO 2016/041635 (24.03.2016 Gazette 2016/12)**

(54) **VERFAHREN UND VORRICHTUNG ZUR VORHERSAGE VON EINEM ODER MEHREREN FÜR DEN AUSGANG EINER BLUTBEHANDLUNG CHARAKTERISTISCHEN PARAMETERN**

METHOD AND DEVICE FOR PREDICTING ONE OR MORE PARAMETERS CHARACTERISTIC OF THE RESULT OF A BLOOD TREATMENT

PROCÉDÉ ET DISPOSITIF DE PRÉVISION D'UN OU DE PLUSIEURS PARAMÈTRES CARACTÉRISTIQUES DU RÉSULTAT D'UN TRAITEMENT SANGUIN

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.09.2014 DE 102014013886**

(43) Veröffentlichungstag der Anmeldung:
**26.07.2017 Patentblatt 2017/30**

(73) Patentinhaber: **Fresenius Medical Care Deutschland GmbH**
**61352 Bad Homburg (DE)**

(72) Erfinder:
• **MILLÁN-GALANTE, Maria**
**61352 Bad Homburg (DE)**
• **DÜLSNER, Erik**
**61197 Florstadt (DE)**
• **WEHMEYER, Wolfgang**
**72076 Tübingen (DE)**

(74) Vertreter: **Herrmann, Uwe**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
**EP-A1- 1 364 666       EP-A1- 2 469 431**
**EP-A2- 1 444 997       WO-A1-2010/112223**
**WO-A1-2014/012670**

• **Uhlin Fredrik ET AL: "Optical Monitoring of Dialysis Dose" In: "Modeling and Control of Dialysis Systems, Volume 2", 1 January 2013 (2013-01-01), Springer, Berlin, XP055878799, ISBN: 978-3-642-27557-9 pages 867-917, DOI: 10.1007/978-3-642-27558-6, Retrieved from the Internet: URL:https://link.springer.com/chapter/10.1 007/978-3-642-27558-6_3>**

**Beschreibung**

Die vorliegende Erfindung betrifft ein Verfahren und eine Vorrichtung zur Vorhersage von einem oder mehreren für den Ausgang einer Blutbehandlung charakteristischen Parametern, wobei es sich bei der Blutbehandlung um eine Behandlung handelt, bei der dem Blut des Patienten über wenigstens eine Membran Flüssigkeit entzogen wird.

Ein wesentliches Ziel bei der Dialyse ist neben der Entfernung von Stoffen aus dem Blut die Entwässerung des Blutes des Patienten. Dialysepflichtige Patienten können mit der Nahrung aufgenommenes Wasser nicht oder nur unzureichend wieder ausscheiden. Dieses Wasser sammelt sich im Patientenblut und im Gewebe des Patienten an, von wo es wieder in das vaskuläre System des Patienten fließen kann.

Durch die im Rahmen der Dialysebehandlung erfolgende Ultrafiltration, d.h. durch den Wasserentzug des Blutes über die Membran des Dialysators wird dieses überschüssige Wasser aus dem Patietenblut entfernt. Das Ultrafiltrationsvolumen wird häufig als ein Maß dafür angenommen, welches Volumen an Flüssigkeit der Dialysepatient zwischen zwei Dialysebehandlungen aufnehmen kann (erlaubtes Trinkvolumen).

Wird das Ultrafiltrationsvolumen als Maß für das erlaubte Trinkvolumen herangezogen, besteht insofern ein Nachteil, als dass es sich dabei um keinen genauen Anhaltspunkt für das erlaubte Trinkvolumen handelt.

Der vorliegenden Erfindung liegt die Aufgabe zugrunde, ein Verfahren und eine Vorrichtung der eingangs genannten Art dahingehend weiterzubilden, dass eine möglichste genaue Vorhersage bzw. Berechnung von Parametern erfolgt, die für den Ausgang einer Blutbehandlung charakteristisch sind.

Diese Aufgabe wird durch ein Verfahren mit den Merkmalen des Anspruchs 1 sowie durch eine Vorrichtung mit den Merkmalen des Anspruchs 8 gelöst. Danach ist vorgesehen, dass es sich bei dem fraglichen Parameter um die Clearance großer Moleküle handelt. Zusätzlich kann es sich bei den fraglichen Parametern auch um das erlaubte Trinkvolumen und/oder um die Über- oder Unterwässerung des Patienten und/oder die erlaubte Salzaufnahme handeln.

Handelt es sich bei dem vorherzusagenden Parameter um das erlaubte Trinkvolumen bis zur nächsten Behandlung oder auch um die Über- oder Unterwässerung, die sich nach der aktuellen bzw. anstehenden Behandlung ergibt, werden diese Parameter erfindungsgemäß auf der Grundlage der geplanten Gewichtsabnahme des Patienten durch Ultrafiltration, der Trinkmenge während der Behandlung, des sowie des durch Rest-Diurese entzogenen Volumens vorhergesagt.

Handelt es sich bei dem vorherzusagenden Parameter um die Clearance großer Moleküle, insbesondere um die Clearance von Mikroglobolin, ist erfindungsgemäß vorgesehen, dass die Vorhersage dieser Clearance basierend auf der Harnstoff-Clearance vorgenommen wird, wobei die Harnstoff-Clearance durch eine Messung der Leitfähigkeit im verbrauchten Dialysat ermittelt wird.

Handelt es sich bei dem vorherzusagenden Parameter um die erlaubte Salzaufnahme bis zur nächsten Dialysebehandlung, erfolgt die Vorhersage dieses Parameters auf der Grundlage der durch Ultrafiltration sowie durch Diffusion aus dem Blut entfernten Natriumionen-Menge.

Durch das erfindungsgemäße Verfahren sowie durch die Vorrichtung gemäß der Erfindung ist es möglich, dem Patienten bzw. dem Nutzer eines Blutbehandlungsgerätes, insbesondere eines Dialysegerätes einen möglichst genauen Anhaltspunkt über den Wert eines oder mehrerer Parameter zu liefern, der sich in Abhängigkeit von Größen, die im Rahmen der Blutbehandlung oder nach deren Abschluss eine Rolle spielen, einstellt bzw. am Ende der Behandlung ergeben wird.

So ist es beispielsweise möglich, mit dem erfindungsgemäßen Verfahren bzw. mit der erfindungsgemäßen Vorrichtung eine vergleichsweise genaue Vorhersage des erlaubten Trinkvolumens bis zur nächsten Behandlung basierend auf den genannten Größen zu liefern.

Somit ist es möglich, die Konsequenz einer Programmierung eines Blutbehandlungsgeräts beispielsweise durch Eingabe der Ultrafiltrationsmenge etc. zu ermitteln und das Ergebnis in geeigneter Weise dem Patienten bzw. dem Nutzer des Gerätes anzuzeigen.

Daraus ergeben sich die Vorteile, dass der Nutzer bzw. das Bedienpersonal noch während der Behandlung oder auch vor der Behandlung eingreifen kann, wenn die vorhergesagten Parameter keine gewünschten Werte annehmen oder außerhalb von Sollwertbereichen liegen.

Die Patienten bzw. Nutzer können unmittelbar nachdem sie in die Programmierung des Blutbehandlungsgerätes eingegriffen bzw. diese vorgenommen haben die Konsequenzen ablesen und ggf. die Einstellungen nochmals korrigieren.

Ein weiterer Vorteil besteht darin, dass der Patient z.B. durch Ausgabe des erlaubten Trinkvolumens bis zur nächsten Blutbehandlung eine höhere Eigenverantwortung und eine Bewusstseinsstärkung der Erkrankung bzw. der Therapie erhält. Auch etwaige Konflikte zwischen Patient und Pflegepersonal werden dadurch behoben, dass durch das Verfahren bzw. durch die Vorrichtung ein objektiver Parameterwert angezeigt wird.

Handelt es sich bei dem vorhergesagten Parameter um das erlaubte Trinkvolumen, kann dieser aus der geplanten Gewichtsabnahme durch Ultrafiltration abzüglich des Trinkvolumens während der Behandlung und abzüglich des Rinseback-Volumens und zuzüglich des durch Rest-Diurese entzogenen Volumens und abzüglich oder zuzüglich eines Volumens berechnet werden, das aufgrund von Eingriffen während der laufenden Behandlung zu- oder abgeführt wird bzw. nicht zu- oder abgeführt wird, wie dies beispielsweise bei einer verringerten Ultrafiltrationsrate der Fall ist.

Das Rinseback-Volumen ist ein Teil eines Flüssigkeitsvolumens an Substituat oder Kochsalzlösung, mit dem am Ende der Behandlung das Blut aus dem extrakorporalen Blutkreislauf zurück in das vaskuläre System des Patienten gedrückt wird. Ein geringer Teil des Substituats bzw. der Kochsalzlösung gelangt hierbei mit in das vaskuläre System bzw. vermischt sich auch mit einem kleinen Teil des Patientenblutes. Bei diesem Teil handelt es sich um das Rinseback-Volumen. Dieses kann beispielsweise abgeschätzt werden oder auch als Information in der Maschinensteuerung hinterlegt sein.

Das durch Rest-Diurese erzeugte Volumen ist das Volumen an Flüssigkeit, das aufgrund der Restnierentätigkeit zwischen zwei Blutbehandlungen aus dem Blut des Patienten entzogen wird.

Aus der Nettobilanz aus Gewichtsverlust bzw. Flüssigkeitsentzug und Gewichtszunahme bzw. Flüssigkeitsaufnahme oder vermindertem Flüssigkeitsentzug errechnet sich die erlaubte Trinkmenge bis zur nächsten Blutbehandlung bzw. bis zur nächsten Dialysebehandlung.

Ein weiterer wichtiger Dialyseparameter ist die Über- bzw. Unterwässerung des Patienten. Diese bestimmt sich aus der Differenz aus dem vorhergesagten Gewicht des Patienten nach der Behandlung und dem Normalgewicht, d.h. der Normohydration des Patienten. Entsprechen sich beide Werte, liegt somit weder eine Über- noch eine Unterwässerung vor.

Das vorhergesagte Gewicht des Patienten nach der Behandlung wird aus dessen Vordialysegewicht abzüglich der geplanten Gewichtsabnahme durch Ultrafiltration zuzüglich eines Trinkvolumens während der Behandlung und zuzüglich des Rinseback-Volumens ermittelt. Abgezogen wird gegebenenfalls noch das durch Rest-Diurese entzogene Volumen. Weiterhin wird das Volumen berücksichtigt, dass aufgrund von Eingriffen währen der laufenden Behandlung zu oder abgeführt wird.

Bei den Eingriffen bzw. Faktoren, die während der laufenden Behandlung auf das Volumen bzw. auf die Flüssigkeitsbilanz einen Einfluss haben, kann es sich beispielsweise um eine Bolusgabe und/oder um eine Änderung der Behandlungsdauer und/oder um eine Änderung des Ultrafiltrationsziels handeln. Ein Beispiel für einen solchen Effekt stellt zum Beispiel die Zeitverkürzung dar, d.h. die Verkürzung der Behandlungsdauer, die bei gleichbleibender Ultrafiltrationsrate zu einem geringeren entzogenen Flüssigkeitsvolumen führt.

Das Rinseback-Volumen und/oder das durch Rest-Diurese gewonnene Volumen kann beispielsweise aus einem Speicher bzw. aus einer Datenbank bezogen werden oder auch abgeschätzt werden. Grundsätzlich kann einer oder beide diese Parameter z.B. als Information in der Maschinensteuerung hinterlegt sein.

Vorstellbar ist es, dass die tatsächliche Gewichtsveränderung bzw. das Gewicht des Patienten und/oder das durch Ultrafiltration entzogene Volumen bzw. die Ultrafiltrationsrate und/oder das Trinkvolumen während der Behandlung durch geeignete Messeinrichtungen erfasst werden.

So ist es möglich, dass das Patientengewicht bzw. die Gewichtsänderung über die Zeit durch eine Waage überwacht wird. Ebenfalls möglich ist die Überwachung der Ultrafiltrationsmenge bzw. -rate durch einen oder mehrere Flusssensoren oder dergleichen. Die Trinkmenge während der Behandlung kann über ein beliebiges Userinterface (beispielsweise ein Smartphone, eine Fernbedienung oder dergleichen) dem Blutbehandlungsgerät bekannt gemacht werden. Denkbar ist auch eine Überwachung des Patienten eine Kamera und die Übertragung der dabei gewonnenen Informationen hinsichtlich des Trinkvolumens an das Dialysegerät.

Ein weiterer informativer Parameter ist die durchschnittliche Überwässerung TAFO (time average fluid overload). Zur Darstellung dieses Wertes wird auf Behandlungsdaten von zurückliegenden Blutbehandlungen zurückgegriffen. Vorzugsweise wird der Wert der mittleren Überwässerung als Mittelwert aus den Werten der Überwässerung vor und nach vorausgegangenen Behandlungen sowie aus dem Wert der Überwässerung vor der aktuellen Behandlung und dem vorhergesagten Wert der Überwässerung nach der aktuellen Behandlung gebildet.

Durch das vorliegende Verfahren lässt sich auch die Clearance großer Moleküle und insbesondere von Mikroglobulin ($\beta$2M) abschätzen. Dabei wird die Harnstoff-Clearance zugrunde gelegt und basierend auf diesem Wert eine Abschätzung der Clearance großer Moleküle vorgenommen. Es ist vorgesehen, dass die Harnstoff-Clearance durch Bestimmung bzw. Messung der Leitfähigkeit im verbrauchten Dialysat ermittelt wird. Ein solches Verfahren ist beispielsweise aus der EP 1444997 B1 bekannt, auf die insoweit Bezug genommen wird. Das für die Abschätzung der Harnstoff-Clearance erforderliche Verteilungsvolumen kann durch Messung der Bioimpedanz von Körperteilen und von daraus abgeleiteten Körperbestandteilen, wie Fett, Wasser und Muskelmasse ermittelt werden.

Ein weiterer wichtiger Parameter ist die Abschätzung der zulässigen Salzaufnahme bis zur nächsten Dialysebzw. Blutbehandlung.

Während der Blutbehandlung bzw. Dialyse wird dem Patienten Salz per Diffusion und per Konvektion bzw. Ultrafiltration über die Membran des Dialysators entzogen. Bis zur nächsten Behandlung sollte der Patient nicht wesentlich mehr als diese Menge an Salz zu sich nehmen. Um die entzogene Salzmenge zu bestimmen kann die aktuelle Plasmakonzentration von Natrium im Blutplasma ermittelt werden. Ein solches Verfahren ist aus der WO 2010/112223 A1 bekannt, auf die soweit Bezug genommen wird. Mit diesem Wert der Plasmakonzentration kann sodann die Natriummenge, die mittels Ultrafiltration über die Membran aus dem Blut entfernt wird, abgeschätzt werden. Ferner kann die Menge an diffusiven Stofftransport von Natriumionen abgeschätzt werden, wenn der Wert über das gesamte Körperwasser und die Differenz aus pre- und postdialytischer Plasmakonzentration berücksichtigt wird.

Wie auch die weiteren Parameter kann die zulässige Salzaufnahme bis zur nächsten Dialyse in geeigneter Weise ausgegeben werden, sodass die Empfehlung des Arztes entsprechend bestärkt wird und der Patient einen objektiven Wert erhält, an dem er sich orientieren kann.

Wie oben ausgeführt, werden der oder die vorhergesagten Parameter und/oder eine oder mehrere der Größen, die die fraglichen Parameter beeinflussen, mittels wenigstens einer Ausgabevorrichtung ausgegeben. Vorzugsweise handelt es sich dabei um einen Monitor und insbesondere um einen Touchscreen-Monitor. Grundsätzlich sind auch andere Ausgabevorrichtungen denkbar und von der Erfindung mit umfasst.

In einer weiteren Ausgestaltung der Erfindung ist vorgesehen, dass eine Änderung des vorhergesagten Wertes des Parameters berechnet und an der Ausgabevorrichtung ausgegeben wird. Nimmt der Nutzer oder der Patient somit beispielsweise eine Änderung einer Größe vor, die auf den vorherzusagenden Parameter Einfluss hat, wie beispielsweise eine Änderung des Ultrafiltrationsvolumens, kann vorgesehen sein, dass die sich daraus ergebende Konsequenz berechnet wird und dem Patienten bzw. Nutzer unmittelbar ausgegeben wird. So ist es beispielsweise möglich, bei einer Änderung des Ultrafiltrationsvolumens unmittelbar dem Patienten anzuzeigen, welche Konsequenzen diese Änderung für das erlaubte Trinkvolumen oder auch für die erlaubte Salzaufnahme bis zur nächsten Behandlung haben kann.

Vorzugsweise besteht die Möglichkeit, dass durch einen Nutzer ein oder mehrere Werte eingegeben werden können, die auf den vorhergesagten Wert des Parameters Einfluss haben.

Somit ist es möglich, dass der Arzt bzw. Patient unmittelbar auf die Vorhersage durch eine veränderte Verschreibung reagiert.

Die vorliegende Erfindung betrifft des Weiteren eine Vorrichtung zur Vorhersage von einem oder mehreren für den Ausgang einer Blutbehandlung charakteristischen Parametern gemäß den Merkmalen des Anspruchs 8.

Weitere bevorzugte Ausgestaltungen der Vorrichtung sind Gegenstand der Unteransprüche 9 bis 14.

Die vorliegende Erfindung betrifft des Weiteren eine Blutbehandlungsvorrichtung insbesondere ein Dialysegerät zur Durchführung einer Blutbehandlung mit einem extrakorporalen Kreislauf, in dem ein Filter bzw. Dialysator angeordnet ist. Dieser wird auf einer Seite von Blut durchströmt und je nach Art der Behandlung auf der anderen Seite von Dialysat, das einen Dialysatkreislauf durchströmt. Über die vorzugsweise als Hohlfaserbündel ausgebildete Membran treten einzelne Stoffe aus dem Blut über und des erfolgt durch Ultrafiltration eine Volumenverringerung bzw. Entwässerung des Patientenblutes.

Das Blutbehandlungsgerät besteht der Vorrichtung gemäß den Ansprüchen 8 bis 14 bzw. weist wenigstens eine derartige Vorrichtung auf.

Weitere Einzelheiten und Vorteile der Erfindung werden anhand eines in der Zeichnung beschriebenen Ausführungsbeispiels näher erläutert. Es zeigen:

Figur 1: eine Darstellung des Körpergewichtes in Abhängigkeit verschiedener Einflussgrößen zur Ermittlung des erlaubten Trinkvolumens bis zur nächsten Behandlung und

Figur 2: eine Darstellung des Körpergewichtes in Abhängigkeit verschiedener Größen zur Ermittlung der Überwässerung des Patienten nach der Behandlung.

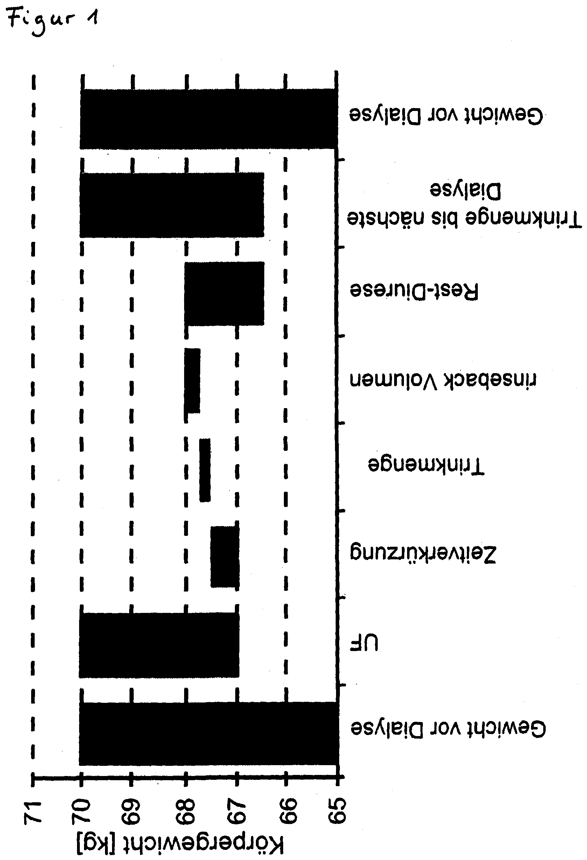

In Figur 1 ist das Körpergewicht eines Dialysepatienten dargestellt sowie verschiedene Größen, die auf das Körpergewicht nach der Dialyse Einfluss haben. Ebenfalls dargestellt ist die erlaubte Trinkmenge bis zur nächsten Dialysebehandlung.

In Figur 1 ist in dem rechten und linken Balken das Patientengewicht vor der Dialyse dargestellt, wobei das untere Ende der Balken im Sinne der Übersichtlichkeit abgeschnitten ist. Wie dies aus Figur 1 hervorgeht, weist der Patient vor der Dialyse ein Körpergewicht von 70 Kg auf. Dem Patienten soll während der Dialyse eine gewisse Menge an Flüssigkeit durch Ultrafiltration entzogen werden, um sein so genanntes Trockengewicht zu erreichen. In dem vorliegenden Beispiel ist die Ultrafiltrationsmenge auf 3 Kg entsprechend 3 Liter Flüssigkeitsentzug eingestellt. Dies ergibt sich aus dem Balken UF gemäß Figur 1.

Das Verhalten des Patienten und etwaige geänderte Parameter während der Dialyse können den Nettoflüssigkeitsentzug aber verändern, so dass die erlaubte Trinkmenge nicht unbedingt dem entzogenen Ultrafiltrationsvolumen entsprechen muss. Wie dies aus Figur 1 hervorgeht spielen neben der geplanten Gewichtsabnahme bei der aktuellen Behandlung (Daten aus der Dialysemaschine) des Weiteren Konsequenzen der in der laufenden Behandlung vorgenommenen Eingriffe z.B. auf die Ultrafiltrationsmenge eine Rolle. Diese Konsequenzen bzw. Eingriffe sind in Figur 1 unter dem Begriff "Zeitverkürzung" zusammengefasst. Neben einer Zeitveränderung kann zum Beispiel eine Bolusgabe oder auch eine Veränderung des Ultrafiltrationsziels darunter fallen. In dem hier dargestellten Beispiel ist die Zeitverkürzung ein Parameter, der zu einer Vergrößerung des Volumens bzw. des Körpergewichtes am Ende der Behandlung führt, da die Zeitverkürzung bei unveränderter Ultrafiltrationsrate eine Verringerung des Ultrafiltrationsvolumens zur Folge hat.

Der Balken "Trinkmenge" verdeutlicht das Volumen an Flüssigkeit, das der Patient während der laufenden Behandlung zu sich nimmt bzw. im Rahmen der anstehenden Behandlung zu sich nehmen wird. Eine andere Quelle von Flüssigkeit ist das Volumen an Substituat oder Kochsalzlösung, das in Figur als Rinseback-Volumen gekennzeichnet ist.

Während die drei Größen "Zeitverkürzung", "Trinkmenge" und "Rinseback-Volumen" in dem hier dargestellten Beispiel zu einer Zunahme des Körpergewichtes bzw.-volumens führen, führt die Rest-Diurese, d.h. die Restnierentätigkeit zu einer Abnahme von Körpergewicht und -volumen bis zur nächsten Behandlung. Aus der Nettobilanz aus Gewichtsverlust durch Flüssigkeitsentzug und Gewichtszunahme durch Flüssigkeitsaufnahme bzw. verminderten Flüssigkeitsentzug über die Dialysedauer errechnet sich die erlaubte Trinkmenge bis zur nächsten Dialyse, die in Figur 1 als 2. Balken von rechts dargestellt ist.

Wie dies aus Figur 1 hervorgeht, erstrecken sich die Balken der Ereignisse (UF und Rest-Diurese), die zu einem Flüssigkeitsentzug des Patienten führen, nach unten. Die Balken der Ereignisse (Zeitverkürzung, Trinkmenge und rinseback Volumen), die zu einer Flüssigkeitszunahme des Patienten führen, erstrecken sich nach oben. Wie aus Figur 1 ersichtlich, sind die einzelnen Ereignisse nebeneinander dargestellt. Dabei werden die Basislinien, von denen aus sich die Balken jeweils erstrecken, (mit Ausnahme des rechten und linken Balkens, die das Gewicht vor der Dialyse darstellen) durch den Endpunkt des jeweils links benachbarten Balkens gebildet. So beginnt der Balken "UF" bei dem Endpunkt des Balkens für das Gewicht des Patienten, d.h. bei der das Gewicht des Patienten repräsentierenden Linie bei 70 kg und verläuft ausgehend davon nach unten, weil es sich bei der Ultrafiltration um einen Flüssigkeitsentzug handelt. Die Strecke, um die sich der Balken UF nach unten erstreckt, entspricht der im Rahmen der Ultrafiltration entzogenen Flüssigkeitsmenge. Eine entsprechende Vorgehensweise gilt für die weiteren Ereignisse. Ausgehend von dem unteren Endpunkt des Balkes UF erstreckt sich der Balken für die Zeitverkürzung. Dieser Balken verläuft nach oben, weil die Zeitverkürzung zu einer Flüssigkeitszunahme bzw. zu weniger entzogener Flüssigkeit führt. An den Endpunkt des Balkens der Zeitverkürzung schließt sich der Balken der Trinkmenge an etc.

Die erlaubte Trinkmenge bis zur nächsten Dialysebehandlung ergibt sich dann einfach aus der Differenz des Endpunktes des ersten oder letzten Balkens (Trockengewicht des Patienten) und dem Endpunkt des letzten Balkens (Rest-Diurese).

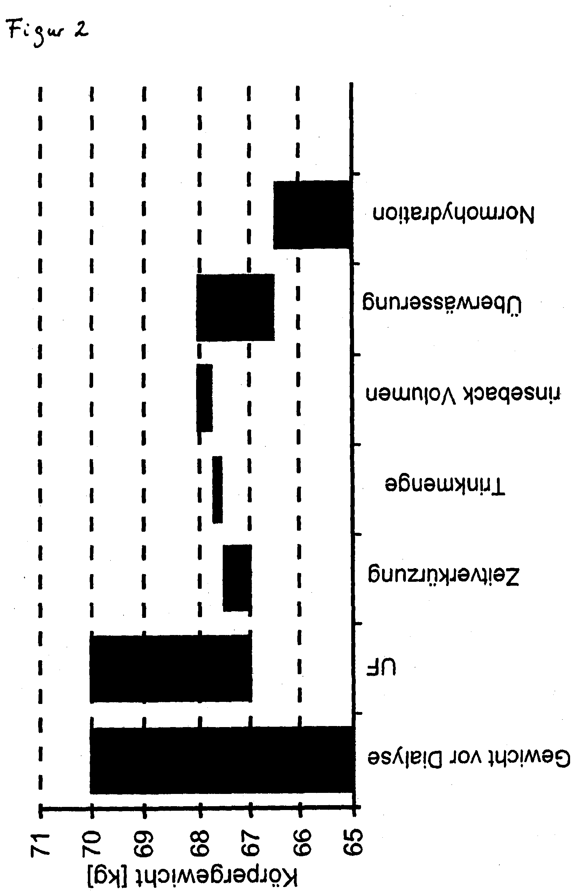

Eine entsprechende Vorgehensweise gilt für die Darstellung gemäß Figur 2.

Grundsätzlich ist es auch möglich und von der Erfindung umfasst, die Balken der Ereignisse, die zu einem Flüssigkeitsentzug des Patienten führen, nach oben und die Balken der Ereignisse, die zu einer Flüssigkeitszunahme des Patienten führen, nach unten verlaufen zu lassen.

Die Trinkmenge, die der Patient während der aktuellen Behandlung zu sich nimmt kann in Form von Daten über die Dialysemaschine oder durch eine Datenbank bereitgestellt werden. Dies gilt entsprechend für das Rinseback-Volumen und auch für die Rest-Diurese. Diese Daten können beispielsweise über die Patientenkarte oder auch über ein Netzwerk aus einer Patientendatenbank ermittelt werden und bereitgestellt werden.

Figur 2 zeigt eine Darstellung des Körpergewichtes in Abhängigkeit von verschiedenen Größen zur Ermittlung des Parameters "Überwässerung". Die Überwässerung stellt für den Arzt eine wichtige Information am Ende der laufenden Dialyse, vor der nächsten Dialyse oder auch als mittlere Überwässerung über zwei oder mehr Dialysebehandlungen dar.

Wie dies aus Figur 2 hervorgeht wird die Überwässerung des Patienten am Ende der laufenden Dialyse aus dem Gewicht vor der Dialyse (Daten der Waage) bzw. von der Patientenkarte oder über ein Netzwerk abzüglich des Normohydrationsgewichtes ermittelt. Dieses lässt sich beispielsweise auch von der Patientenkarte oder über ein Netzwerk beziehen.

Erfolgt im Rahmen der Behandlung ein Netto-Flüssigkeitsentzug in einem Ausmaß, so dass am Ende der Behandlung das Normohydrationsgewicht erreicht wird, liegt keine Überwässerung vor.

Für die Berechnung, wieviel Körperflüssigkeit entzogen wird, spielen in dem hier aufgeführten Ausführungsbeispiel die Ultrafiltrationsrate, die Zeitverkürzung bzw. sonstige besondere Effekte, die Trinkmenge und das Rinseback-Volumen eine Rolle. Abgesehen von der Rest-Diurese werden somit dieselben Größen zugrunde gelegt, wie bei dem Ausführungsbeispiel gemäß Figur 1, sodass entsprechend Bezug genommen wird.

Aus dem Gewicht vor der Dialyse und dem voraussichtlichen Flüssigkeitsentzug abzüglich der Normohydration ergibt sich die Überwässerung, die in Figur 2 als zweiter Balken von rechts dargestellt ist. Entsprechend kann sich je nach entzogener Flüssigkeitsmenge auch eine Unterwässerung des Patienten am Ende der Dialyse ergeben.

Die Überwässerung bzw. Unterwässerung kann als Absolutwert dargestellt werden z.B. in Liter, oder auch nach Division durch das Normohydrationsgewicht als relative Überwässerung oder Unterwässerung in Prozent. Üblicherweise bezieht sich die Überwässerung auf den Wert relativ zur Normohydration und ist im Allgemeinen nach Verschreibung des Arztes (Trockengewicht, Gewicht nach Dialyse) nicht null. Positive oder negative Werte sind möglich. Die Überwässerung bzw. Unterwässerung wird wesentlich durch die Kreislaufstabilisierung des Patienten gegen Ende der Dialyse bestimmt.

Ein weiterer informativer Parameter, der erfindungsgemäß ausgegeben werden kann ist die durchschnittliche Überwässerung TAFO. Dieser Wert hilft dem Arzt, die mittlere kardiovaskuläre Belastung des Körpers durch Überwässerung einzuschätzen. Denkbar ist es, die Ermittlung der Überwässerung über ein 7-Tage-Intervall, d.h. im Regelfall über die letzten drei Dialysebehandlungen vorzunehmen:

Im Einzelnen ergibt sich in diesem Fall

$$TAFO = 1/6 * [(ÜW1pre + ÜW2pre + ÜW3pre) + (ÜW1post + ÜW2post + ÜW3post)]$$

Dabei stellen die Größen ÜW1pre, ÜW2pre die Überwässerung vor der Dialyse von vorausgegangenen Dialysebehandlungen 1 und 2 dar und die Werte ÜW1post und ÜW2post die Überwässerungswerte nach der Dialyse dieser vorausgegangenen Behandlungen 1 und 2. Die Daten können aus Datenbankinformation z.B. aus der Patientenkarte, über ein Netzwerk oder aus einem internen Speicher der Dialysemaschine bezogen werden.

In dem hier dargestellten Beispiel betrifft der Wert ÜW3pre die Größe der Überwässerung, die sich aus dem Gewicht vor der aktuellen Dialyse, erhalten beispielsweise durch eine Messung durch eine Waage, abzüglich der Normohydration ergibt. Der Wert ÜW3post ist die Vorhersage der Überwässerung für die aktuelle Behandlung, die wie zuvor beschrieben errechnet bzw. vorhergesagt werden kann. Der tatsächliche Ist-Wert von ÜW3post kann nach der Behandlung in einer Datenbank abgelegt werden um für die folgenden Berechnungen der mittleren Überwässerung zur Verfügung zu stehen.

Die Messung der Clearance von großen Molekülen, wie Mikroglobolin wird erfindungsgemäß teils durch gemessene Werte und teils durch gespeicherte Werte bestimmt. Ausgangspunkt für die Abschätzung der mittleren Clearance großer Moleküle ist eine Abschätzung der Filterleistung für Harnstoff. Der technische Zusammenhang zwischen der Harnstoff-Clearance und der Clearance großer Moleküle ist in Kennfeldern abgelegt und daher bekannt. Daraus kann zunächst die relative Performance der Clearance großer Moleküle zur Harnstoff-Clearance abgeschätzt werden.

Zur Berechnung der Harnstoff-Clearance kann auf bekannte Verfahren zurückgegriffen werden, wie beispielsweise auf die Messung der Leitfähigkeit im verbrauchten Dialysat. Insofern wird auf die EP 1 444 997 B1 Bezug genommen. Dabei wird die Messung der Clearance oder der Dialysanz von Harnstoff auf der Dialysatseite vorgenommen.

Weitere Parameter, die in diese Berechnung einfließen können, können Flussparamater der aktuellen Behandlung sein, wie beispielsweise der Blutfluss, der Dialysatfluss, die Ultrafiltrationsrate und bei der Hämodiafiltration bzw. Hämofiltration der Infusionsfluss. Weitere Parameter sind der Behandlungsmodus (Hämodialyse, Hömodiafiltration und Hämofiltration) und die Produktdaten des Blutfilters bzw. Dialysators.

Sobald die Harnstoff-Clearance bestimmt wurde kann eine "Kalibrierung" auf die tatsächlichen Betriebsbedingungen erfolgen. Aus den programmierten Daten für die Flüsse lässt sich eine Vorhersage über die mittlere Clearance von Harnstoff bis zum Ende der Dialyse treffen. Hierbei fließt auch die Vorhersage der Infusionsvolumina mit ein. Mittels Kalibrierkurven bzw. Kennfeldern kann dann basierend auf der mittleren Clearance von Harnstoff auf eine mittlere Clearance von großen Molekülen, insbesondere von Mikroglobolin hochgerechnet bzw. geschlossen werden. Auch dieser Wert kann entsprechend angezeigt werden, und es ist möglich, durch Änderungen bestimmter Parameter, wie beispielsweise durch die Änderungen von Flussraten Einfluss auf die Clearance zu nehmen und sich den geänderten Wert ebenfalls anzeigen zu lassen.

Fehler in der Kalkulation können dadurch entstehen, dass der Zusammenhang von Harnstoff-Clearance und der Clearance großer Moleküle durch die spezielle Zusammensetzung des Blutes eines Patienten variabel ist. Um diese Problematik zu lindern bzw. zu beheben sind folgende Korrekturparameter vorstellbar:

Konzentration von Gerinnungsfaktoren, Hämatokrit, Albumin und Eiweiß im Blut (Daten aus der Blutanalyse des Patienten),

Erhöhung der Konzentration von Gerinnungsfaktoren, Hämatokrit, Albumin und Eiweiß während der Dialyse durch Eindicken des Blutes als Folge der Ultrafiltration (Daten aus Blutvolumensensor während der Behandlung).

Die Darstellung des Ergebnisses, d.h. die Vorhersage der mittleren Clearance großer Moleküle kann beispielsweise als mittlere Clearance erfolgen, oder auch Leistungswert in Form des Produktes aus Clearance und Zeit, sowie auch als relativer Leistungswert in Form des Produktes aus Clearance und Zeit dividiert durch das Verteilungsvolumen der großen Moleküle. Dieses lässt sich wie oben ausgeführt durch die Messung der Bioimpedanz von Körperteilen und von daraus abgeleiteten Körperbestandteilen wie Fett, Wasser und Muskelmasse bestimmen.

Durch die vorliegende Erfindung ist möglich, dem Arzt bzw. dem Nutzer des Gerätes anzuzeigen, welchen Wert für die Behandlung wesentliche Parameter nach der Behandlung annehmen können. Diese Vorhersage ermöglicht es, umgehend einzugreifen, sofern unerwünschte Werte erhalten werden.

Außer einer Darstellung auf einem Bildschirm sind auch beliebige andere Darstellungs- bzw. Ausgabeoptionen, wie z.B. eine akustische Sprachausgabe oder ein Ausdruck denkbar.

Sowohl für die Trinkmenge als auch für die Salzaufnahme wird bislang davon ausgegangen, dass der Patientenstatus bei der nächsten Dialyse derselbe sein sollte, wie bei der aktuellen Blutbehandlung. Es besteht jedoch die Möglichkeit, dass der Arzt bezüglich der Überwässerung eine leichte Korrektur vornehmen möchte. Nach dem derzeitigen Stand der Technik wird diese Korrektur bei der nächsten Dialyse durch eine Herabsetzung des Zielgewichtes angestrebt, was eine höhere Ultrafiltrationsmenge und damit eine größere Belastung des Körpers mit sich bringt.

Gemäß der vorliegenden Erfindung kann diese Korrektur, beispielsweise ein Volumenverlust von 200 ml bereits vor der aktuellen Dialyse vom Arzt festgelegt werden und auf geeignete Weise durch Eingabemittel an das Dialysegerät übertragen werden. Diese Korrektur kann dann in die Darstellung der zulässigen Trinkmenge mit einfließen, nämlich in Form der errechneten Trinkmenge abzüglich der Korrektur. In dem Fall ergibt sich als zulässiges Trinkvolumen die errechnete Trinkmenge abzüglich 200 ml. Entsprechendes gilt auch für die Salzaufnahme.

**Patentansprüche**

1. Verfahren zur Vorhersage von einem oder mehreren für den Ausgang einer Blutbehandlung charakteristischen Parametern, wobei es sich bei der Blutbehandlung um eine Behandlung handelt, bei der dem Blut des Patienten über wenigstens eine Membran Flüssigkeit entzogen wird, **dadurch gekennzeichnet, dass** der Parameter die Clearance großer Moleküle ist, wobei die Vorhersage der Clearance großer Moleküle basierend auf der Harnstoff-Clearance vorgenommen wird, wobei die Harnstoff-Clearance durch eine Messung der Leitfähigkeit im verbrauchten Dialysat ermittelt wird.

2. Verfahren nach Anspruch 1, **dadurch gekennzeichnet, dass** es sich dem oder den weiteren für den Ausgang eine Blutbehandlung charakteristischen Parametern um das erlaubte Trinkvolumen, die Über-oder Unterwässerung des Patienten und/oder um die erlaubte Salzaufnahme handelt, wobei die Vorhersage des erlaubten Trinkvolumens sowie die Vorhersage der Über- oder Unterwässerung basierend auf der geplanten Gewichtsabnahme durch Ultrafiltration, der Trinkmenge während der Behandlung, des Rinseback-Volumens und von Rest-Diurese-Daten vorgenommen wird und/oder wobei die Vorhersage der erlaubten Salzaufnahme basierend auf der durch Ultrafiltration sowie durch Diffusion aus dem Blut entfernten Natriumionen-Menge erfolgt.

3. Verfahren nach Anspruch 2, **dadurch gekennzeichnet, dass** das vorhergesagte erlaubte Trinkvolumen aus der geplanten Gewichtsabnahme durch Ultrafiltration abzüglich des Trinkvolumens während der Behandlung abzüglich des Rinseback-Volumens zuzüglich des durch Rest-Diurese entzogenen Volumens und abzüglich oder zuzüglich von Volumen berechnet wird, das aufgrund von Eingriffen während der laufenden Behandlung zu- oder abgeführt wird.

4. Verfahren nach Anspruch 2 oder 3, **dadurch gekennzeichnet, dass** die vorhergesagte Über- oder Unterwässerung am Ende der Behandlung aus der Differenz aus dem vorhergesagten Gewicht des Patienten nach der Behandlung und dem Normalgewicht (Normohydration) des Patienten berechnet wird, wobei das vorhergesagte Gewicht des Patienten nach der Behandlung aus dem Vordialysegewicht abzüglich der geplanten Gewichtsabnahme durch Ultrafiltration zuzüglich des Trinkvolumens während der Behandlung zuzüglich des Rinseback-Volumens abzüglich des durch Rest-Diurese entzogenen Volumens und abzüglich oder zuzüglich von Volumen berechnet wird, das aufgrund von Eingriffen während der laufenden Behandlung zu- oder abgeführt wird.

5. Verfahren nach Anspruch 3 oder 4, **dadurch gekennzeichnet, dass** es sich bei den Eingriffen um eine Bolusgabe, um eine Änderung der Behandlungsdauer und/oder um eine Änderung des Ultrafiltrationsziels handelt.

6. Verfahren nach einem der Ansprüche 2 bis 5, **dadurch gekennzeichnet, dass** es sich bei der Vorhersage der Überwässerung um die Vorhersage der mittleren Überwässerung (TAFO) handelt, die Werte der Überwässerung vorausgegangener Behandlungen mit umfasst, wobei vorzugsweise vorgesehen ist, dass die mittlere Überwässerung nach der Beziehung

$$TAFO = 1/n * [(\ddot{U}W1,pre + \ddot{U}W(n-1),pre + \ddot{U}Wn,pre) + (\ddot{U}W1,post + \ddot{U}W(n-1),post + \ddot{U}Wn,post)]$$

berechnet wird, wobei es sich bei ÜW1,pre und ÜW(n-1),pre und bei ÜW1,post und ÜW(n-1),post um Werte der Überwässerung vor bzw. nach vorausgegangenen Behandlungen 1 ... (n-1) handelt, wobei es sich bei ÜWn,pre um den Wert der Überwässerung vor der aktuellen Behandlung ermittelt aus der Differenz aus dem Patientengewicht und der Normohydration handelt und wobei es sich bei ÜWn,post um den vorhergesagten Wert der Überwässerung nach der aktuellen Behandlung handelt.

7. Verfahren nach einem der vorhergehenden Ansprüche, **dadurch gekennzeichnet, dass** die Ermittlung der Harnstoff-Clearance durch Messung der Harnstoffkonzentration im Dialysat oder durch Messung der Leitfähigkeit des Dialysats erfolgt und/oder dass der Wert des oder der vorhergesagten Parameter und/oder eine oder mehrere von den diesen beeinflussenden Größen mittels wenigstens einer Ausgabevorrichtung ausgegeben wird, wobei es sich bei Ausgabevorrichtung vorzugsweise um einen Monitor und insbesondere um einen Touchscreen-Monitor handelt.

8. Vorrichtung zur Vorhersage von einem oder mehreren für den Ausgang einer Blutbehandlung charakteristischen Parametern, wobei es sich bei der Blutbehandlung um eine Behandlung handelt, bei der dem Blut des Patienten über wenigstens eine Membran Flüssigkeit entzogen wird, **dadurch gekennzeichnet, dass** es sich bei dem Parameter um die Clearance großer Moleküle handelt, wobei die Vorrichtung Berechnungsmittel aufweist, die derart ausgebildet sind, dass diese die Vorhersage der Clearance großer Moleküle basierend auf der Harnstoff-Clearance vornehmen.

9. Vorrichtung nach Anspruch 8, **dadurch gekennzeichnet, dass** es sich bei dem oder den weiteren für den Ausgang einer Blutbehandlung charakteristischen Parametern um das erlaubte Trinkvolumen, die Über- oder Unterwässerung des Patienten und/oder um die erlaubte Salzaufnahme handelt, wobei die Vorrichtung Berechnungsmittel aufweist, die derart ausgebildet sind, dass diese die Vorhersage des erlaubten Trinkvolumens sowie die Vorhersage der Über- oder Unterwässerung basierend auf der geplanten Gewichtsabnahme durch Ultrafiltration, der Trinkmenge während der Behandlung, des Rinseback-Volumens und von Rest-Diurese-Daten vornehmen und/oder dass diese die Vorhersage der erlaubten Salzaufnahme basierend auf der durch Ultrafiltration sowie durch Diffusion aus dem Blut entfernten Natriumionen-Menge vornehmen.

10. Vorrichtung nach Anspruch 9, **dadurch gekennzeichnet, dass** die Berechnungsmittel derart ausgebildet sind, dass das vorhergesagte erlaubte Trinkvolumen aus der geplanten Gewichtsabnahme durch Ultrafiltration abzüglich des Trinkvolumens während der Behandlung abzüglich des Rinseback-Volumens zuzüglich des durch Rest-Diurese entzogenen Volumens und abzüglich oder zuzüglich von Volumen berechnet wird, das aufgrund von Eingriffen während der laufenden Behandlung zu- oder abgeführt wird.

11. Vorrichtung nach Anspruch 9 oder 10, **dadurch gekennzeichnet, dass** die Berechnungsmittel derart ausgebildet sind, dass die vorhergesagte Über- oder Unterwässerung am Ende der Behandlung aus der Differenz aus dem vorhergesagten Gewicht des Patienten nach der Behandlung und dem Normalgewicht (Normohydration) des Patienten berechnet wird, wobei das vorhergesagte Gewicht des Patienten nach der Behandlung aus dem Vordialysegewicht abzüglich der geplanten Gewichtsabnahme durch Ultrafiltration zuzüglich des Trinkvolumens während der Behandlung zuzüglich des Rinseback-Volumens abzüglich des durch Rest-Diurese entzogenen Volumens und abzüglich oder zuzüglich von Volumen berechnet wird, das aufgrund von Eingriffen während der laufenden Behandlung zu- oder abgeführt wird.

12. Vorrichtung nach Anspruch 10 oder 11, **dadurch gekennzeichnet, dass** es sich bei den Eingriffen um eine Bolusgabe, um eine Änderung der Behandlungsdauer und/oder um eine Änderung des Ultrafiltrationsziels handelt.

13. Vorrichtung nach einem der Ansprüche 9 bis 12, **dadurch gekennzeichnet, dass** die Berechnungsmittel derart ausgebildet sind, dass diese den Wert der mittleren Überwässerung (TAFO) berechnen, wobei in die Berechnung Werte der Überwässerung vorausgegangener Behandlungen mit einfließen, wobei vorzugsweise vorgesehen ist, dass die Berechnungsmittel derart ausgebildet sind, dass die mittlere Überwässerung nach der Beziehung

$$TAFO = 1/n * [(ÜW1,pre + ÜW(n-1),pre + ÜWn,pre) + (ÜW1,post + ÜW(n-1),post + ÜWn,post)]$$

berechnet wird, wobei es sich bei ÜW1,pre und ÜW(n-1),pre und bei ÜW1,post und ÜW(n-1)post um Werte der Überwässerung vor bzw. nach vorausgegangenen Behandlungen 1 ... (n-1) handelt, wobei es sich bei ÜWn,pre um den Wert der Überwässerung vor der aktuellen Behandlung ermittelt aus der Differenz aus dem Patientengewicht und der Normohydration handelt und wobei es sich bei ÜWn,post um den vorhergesagten Wert der Überwässerung nach der aktuellen Behandlung handelt.

14. Vorrichtung nach einem der Ansprüche 8 bis 13, **dadurch gekennzeichnet, dass** die Berechnungsmittel derart ausgebildet sind, dass die Ermittlung der Harnstoff-Clearance basierend auf einem oder mehreren Messwerten der Harnstoffkonzentration im Dialysat oder der Leitfähigkeit des Dialysats erfolgt und/oder **dadurch gekennzeichnet, dass** die Vorrichtung wenigstens eine Ausgabevorrichtung aufweist, die derart ausgebildet ist, das der Wert des oder der vorhergesagten Parameter und/oder eine oder mehrere von den diesen beeinflussenden Größen mittels der Ausgabevorrichtung ausgegeben wird, wobei es sich bei Ausgabevorrichtung vorzugsweise um einen Monitor und insbesondere um einen Touchscreen-Monitor handelt..

15. Blutbehandlungsvorrichtung, insbesondere Dialysegerät, zur Durchführung einer Blutbehandlung, bei der dem Blut des Patienten über wenigstens eine Membran Flüssigkeit entzogen wird, **dadurch gekennzeichnet, dass** die Blutbehandlungsvorrichtung wenigstens eine Vorrichtung gemäß einem der Ansprüche 9 bis 14 aufweist oder durch wenigstens eine Vorrichtung gemäß einem der Ansprüche 9 bis 14 gebildet wird.

## Claims

1. A method for predicting one or more parameters characteristic for the outcome of a blood treatment, wherein the blood treatment is a treatment in which the blood of the patient has fluid removed via at least one membrane, **characterized in that** the parameter is the clearance of large molecules with the prediction of the clearance of large molecules being carried out on the basis of the urea clearance, with the urea clearance being determined by a measurement of the conductivity in the used dialyzate.

2. A method in accordance with claim 1, **characterized in that** the further parameter(s) characteristic for the outcome of a blood treatment is/are the allowed drinking volume, the hyperhydration or hypohydration of the patient, and/or the allowed salt intake, with the prediction of the allowed drinking volume and the prediction of the hyperhydration or hypohydration being carried out on the basis of the planned weight loss due to ultrafiltration, of the drinking quantity during the treatment, of the rinseback volume, and of residual diuresis data, and/or with the prediction of the allowed salt intake taking place based on the sodium ion quantity removed from the blood by ultrafiltration and by diffusion.

3. A method in accordance with claim 2, **characterized in that** the predicted allowed drinking volume is calculated from the planned weight loss due to ultrafiltration less the drinking volume during the treatment less the rinseback volume plus the volume removed by residual diuresis and less or plus volume which is led in or led off due to interventions during the ongoing treatment.

4. A method in accordance with claim 2 or claim 3, **characterized in that** the predicted hyperhydration or hypohydration at the end of the treatment is calculated from the difference of the predicted weight of the patient after the treatment and the normal weight (normohydration) of the patient, with the predicted weight of the patient after the treatment being calculated from the predialysis weight less the planned weight loss due to ultrafiltration plus the drinking volume during the treatment plus the rinseback volume less the volume removed by residual diuresis and less or plus volume which is led in or led off due to interventions during the ongoing treatment.

5. A method in accordance with claim 3 or claim 4, **characterized in that** the interventions are a bolus administration, a change of the treatment duration and/or a change of the ultrafiltration target.

6. A method in accordance with one of the claims 2 to 5, **characterized in that** the prediction of the hyperhydration is the prediction of the average hyperhydration (TAFO) which also covers values of the hyperhydration of preceding treatments, with provision preferably being made that the average hyperhydration is calculated according to the relationship

$$TAFO = 1/n * [(\ddot{U}W1,pre + \ddot{U}W(n-1),pre + \ddot{U}Wn,pre) + (\ddot{U}W1,post + \ddot{U}W(n-1),post + \ddot{U}Wn,post)]$$

where UW1,pre and UW(n-1),pre and UW1,post and ÜW(n-1)post are values of the hyperhydration before and after preceding treatments 1 ... (n-1) respectively, where ÜWn,pre is the value of the hyperhydration before the current treatment determined from the difference of the patient's weight and the normohydration, and where UWn,post is the predicted value of the hyperhydration after the current treatment.

7. A method in accordance with one of the preceding claims, **characterized in that** the determination of the urea clearance takes place by measurement of the urea concentration in the dialyzate or by measurement of the conductivity of the dialyzate; and/or **in that** the value of the predicted parameter(s) and/or one or more of the values influencing it is output by means of at least one output apparatus, with the output apparatus preferably being a monitor and in particular being a touchscreen monitor.

8. An apparatus for predicting one or more parameters characteristic for the outcome of a blood treatment, wherein the blood treatment is a treatment in which the blood of the patient has fluid removed via at least one membrane, **characterized in that** the parameter is the clearance of large molecules, with the apparatus having calculation means that are configured such they carry out the prediction of the clearance of large molecules on the basis of the urea clearance.

9. An apparatus in accordance with claim 8, **characterized in that** the further parameter(s) characteristic for the outcome of a blood treatment is/are the allowed drinking volume, the hyperhydration or hypohydration of the patient, and/or the allowed salt intake, with the apparatus having calculation means that are configured such that they carry out the prediction of the allowed drinking volume and the prediction of the hyperhydration or hypohydration on the basis of the planned weight loss due to ultrafiltration, of the drinking quantity during the treatment, of the rinseback volume, and of residual diuresis data; and/or **in that** they carry out the prediction of the allowed salt intake based on the sodium ion quantity removed from the blood by ultrafiltration and by diffusion.

10. An apparatus in accordance with claim 9, **characterized in that** the calculation means are configured such that the predicted allowed drinking volume is calculated from the planned weight loss due to ultrafiltration less the drinking volume during the treatment less the rinseback volume plus the volume removed by residual diuresis and less or plus volume which is led in or led off due to interventions during the ongoing treatment.

11. An apparatus in accordance with claim 9 or claim 10, **characterized in that** the calculation means are configured such that the predicted hyperhydration or hypohydration at the end of the treatment is calculated from the difference of the predicted weight of the patient after the treatment and the normal weight (normohydration) of the patient, with the predicted weight of the patient after the treatment being calculated from the predialysis weight less the planned weight loss due to ultrafiltration plus the drinking volume during the treatment plus the rinseback volume less the volume removed by residual diuresis and less or plus volume which is led in or led off due to interventions during the ongoing treatment.

12. An apparatus in accordance with claim 10 or claim 11, **characterized in that** the interventions are a bolus administration, a change of the treatment duration and/or a change of the ultrafiltration target.

13. An apparatus in accordance with one of the claims 9 to 12, **characterized in that** the calculation means are configured such that they calculate the value of the average hyperhydration (TAFO), with values of the hyperhydration of preceding treatments also entering into the calculation, with provision preferably being made that the calculation means are configured such that the average hyperhydration is calculated in accordance with the relationship

$$TAFO = 1/n * [(ÜW1,pre + ÜW(n-1),pre + ÜWn,pre) + (ÜW1,post + ÜW(n-1),post + ÜWn,post)]$$

where ÜW1,pre and ÜW(n-1),pre and ÜW1,post and ÜW(n-1)post are values of the hyperhydration before and after preceding treatments 1 ... (n-1) respectively, where ÜWn,pre is the value of the hyperhydration before the current treatment determined from the difference of the patient's weight and the normohydration, and where UWn,post is the predicted value of the hyperhydration after the current treatment.

14. An apparatus in accordance with one of the claims 8 to 13, **characterized in that** the calculation means are configured such that the determination of the urea clearance takes place on the basis of one or more measured values of the urea concentration in the dialyzate or of the conductivity of the dialyzate; and/or **characterized in that** the apparatus has at least one output apparatus which is configured such that the value of the predicted parameter(s) and/or one or more of the values influencing it is output by means of the output apparatus, with the output apparatus preferably being a monitor and in particular being a touchscreen monitor.

15. A blood treatment apparatus, in particular a dialysis device, for carrying out a blood treatment, in which the blood of the patient has fluid removed via at least one membrane, **characterized in that** the blood treatment apparatus has at least one apparatus in accordance with one of the claims 9 to 14 or is formed by at least one apparatus in accordance with one of the claims 9 to 14.

**Revendications**

1. Procédé de prévision d'un ou de plusieurs paramètres caractéristiques du résultat d'un traitement sanguin, le traitement sanguin étant un traitement selon lequel du liquide est extrait du sang du patient par le biais d'au moins une membrane, **caractérisé en ce que** le paramètre est la clairance de grandes molécules, la prévision de la clairance de grandes molécules étant réalisée sur la base de la clairance de l'urée, la clairance de l'urée étant déterminée par une mesure de la conductivité dans le dialysat usagé.

2. Procédé selon la revendication 1, **caractérisé en ce que** le ou les autres paramètres caractéristiques du résultat d'un traitement sanguin sont le volume de boisson autorisé, la sur-hydratation ou sous-hydratation du patient et/ou la consommation de sel autorisée, la prévision du volume de boisson autorisé ainsi que la prévision de la sur-hydratation ou sous-hydratation étant réalisée sur la base de la perte de poids par ultrafiltration planifiée, de la quantité bue pendant le traitement, du volume de rinçage de retour et de données relatives à la diurèse résiduelle et/ou la prévision de la consommation de sel autorisée étant effectuée sur la base de la quantité d'ions sodium éliminés du sang par ultrafiltration ainsi que par diffusion.

3. Procédé selon la revendication 2, **caractérisé en ce que** le volume de boisson autorisé prévu est calculé à partir de la perte de poids par ultrafiltration planifiée moins le volume bu pendant le traitement, moins le volume de rinçage de retour, plus le volume extrait par diurèse résiduelle et moins ou plus le volume qui est alimenté ou éliminé en raison d'une intervention au cours du traitement.

4. Procédé selon la revendication 2 ou 3, **caractérisé en ce que** la sur-hydratation ou sous-hydratation prévue est calculée à la fin du traitement à partir de la différence entre le poids prévu du patient après le traitement et le poids normal (normohydratation) du patient, le poids prévu du patient après le traitement étant calculé à partir du poids avant dialyse moins la perte de poids par ultrafiltration prévue, plus le volume bu pendant le traitement, plus le volume de rinçage de retour, moins le volume extrait par diurèse résiduelle et moins ou plus le volume qui est alimenté ou éliminé en raison d'interventions au cours du traitement.

5. Procédé selon la revendication 3 ou 4, **caractérisé en ce que** les interventions sont l'administration d'un bolus, une modification de la durée de traitement et/ou une modification de l'objectif d'ultrafiltration.

6. Procédé selon l'une des revendications 2 à 5, **caractérisé en ce que** la prévision de la sur-hydratation est la prévision de la sur-hydratation moyenne (TAFO), qui comprend également des valeurs de la sur-hydratation de traitements précédents, la sur-hydratation moyenne étant de préférence prévue calculée selon la relation

$$TAFO = 1/n * [(\text{ÜW1,pre} + \text{ÜW(n-1),pre} + \text{ÜWn,pre}) + (\text{ÜW1,post} + \text{ÜW(n-1),post} + \text{ÜWn,post})]$$

où ÜW1,pre et ÜW(n-1),pre et ÜW1,post et ÜW(n-1),post sont des valeurs de la sur-hydratation respectivement avant et après des traitements précédentes 1 ... (n-1), ÜWn,pre étant la valeur de la sur-hydratation avant le traitement actuel déterminée à partir de la différence entre le poids du patient et la normohydratation et ÜWn,post étant la valeur prévue de la sur-hydratation après le traitement actuel.

7. Procédé selon l'une des revendications précédentes, **caractérisé en ce que** la détermination de la clairance de l'urée est effectuée par mesure de la concentration de l'urée dans le dialysat ou par mesure de la conductivité du dialysat et/ou **en ce que** la valeur du ou des paramètres prévus et/ou une ou plusieurs des grandeurs ayant une influence sur ceux-ci sont sorties au moyen d'au moins un dispositif de sortie, le dispositif de sortie étant de préférence un écran et en particulier un écran tactile.

8. Dispositif de prévision d'un ou de plusieurs paramètres caractéristiques du résultat d'un traitement sanguin, le traitement sanguin étant un traitement selon lequel du liquide est extrait du sang du patient par le biais d'au moins une membrane, **caractérisé en ce que** le paramètre est la clairance de grandes molécules, le dispositif comportant des moyens de calcul, qui sont conçus de manière à effectuer la prévision de la clairance de grandes molécules sur la base de la clairance de l'urée.

9. Dispositif selon la revendication 8, **caractérisé en ce que** le ou les autres paramètres caractéristiques du résultat d'un traitement sanguin sont le volume de boisson autorisé, la sur-hydratation ou sous-hydratation du patient et/ou la consommation de sel autorisée, le dispositif comportant des moyens de calcul, qui sont conçus de manière à réaliser la prévision du volume de boisson autorisé ainsi que la prévision de la sur-hydratation ou sous-hydratation sur la base de la perte de poids par ultrafiltration planifiée, de la quantité bue pendant le traitement, du volume de rinçage de retour et de données relatives à la diurèse résiduelle et/ou **en ce que** ceux-ci réalisent la prévision de la consommation de sel autorisée sur la base de la quantité d'ions sodium éliminés du sang par ultrafiltration ainsi que par diffusion.

10. Dispositif selon la revendication 9, **caractérisé en ce que** les moyens de calcul sont conçus de telle manière que le volume de boisson autorisé prévu est calculé à partir de la perte de poids par ultrafiltration planifiée moins le volume bu pendant le traitement, moins le volume de rinçage de retour, plus le volume extrait par diurèse résiduelle et moins ou plus le volume qui est alimenté ou éliminé en raison d'interventions au cours du traitement.

11. Dispositif selon la revendication 9 ou 10, **caractérisé en ce que** les moyens de calcul sont conçus de telle manière que la sur-hydratation ou sous-hydratation prévue est calculée à la fin du traitement à partir de la différence entre le poids prévu du patient après le traitement et le poids normal (normohydratation) du patient, le poids prévu du patient après le traitement étant calculé à partir du poids avant dialyse moins la perte de poids par ultrafiltration prévue, plus le volume bu pendant le traitement, plus le volume de rinçage de retour, moins le volume extrait par diurèse résiduelle et moins ou plus le volume qui est alimenté ou éliminé en raison d'interventions au cours du traitement.

12. Dispositif selon la revendication 10 ou 11, **caractérisé en ce que** les interventions sont l'administration d'un bolus, une modification de la durée de traitement et/ou une modification de l'objectif d'ultrafiltration.

13. Dispositif selon l'une des revendications 9 à 12, **caractérisé en ce que** les moyens de calcul sont conçus de manière à calculer la valeur de la sur-hydratation moyenne (TAFO), des valeurs de la sur-hydratation de traitements précédents étant prises en compte dans le calcul, les moyens de calcul étant de préférence prévus conçus de telle manière que la sur-hydratation moyenne est calculée selon la relation

$$TAFO = 1/n * [(\ddot{U}W1,pre + \ddot{U}W(n\text{-}1),pre + \ddot{U}Wn,pre) + (\ddot{U}W1,post + \ddot{U}W(n\text{-}1),post + \ddot{U}Wn,post)]$$

où ÜW1,pre et ÜW(n-1),pre et ÜW1 ,post et ÜW(n-1)post sont des valeurs de la sur-hydratation respectivement avant et après des traitements précédents 1 ... (n-1), ÜWn,pre étant la valeur de la sur-hydratation avant le traitement actuel déterminée à partir de la différence entre le poids du patient et la normohydratation et ÜWn,post étant la valeur prévue de la sur-hydratation après le traitement actuel.

14. Dispositif selon l'une des revendications 8 à 13, **caractérisé en ce que** les moyens de calcul sont conçus de telle manière que la détermination de la clairance de l'urée est effectuée sur la base d'une ou de plusieurs valeurs de mesure de la concentration de l'urée dans le dialysat ou de la conductivité du dialysat et/ou **caractérisé en ce que** le dispositif comporte au moins un dispositif de sortie, qui est conçu de telle manière que la valeur du ou des paramètres prévus et/ou une ou plusieurs des grandeurs ayant une influence sur ceux-ci sont sorties au moyen du dispositif de sortie, le dispositif de sortie étant de préférence un écran et en particulier un écran tactile.

15. Dispositif de traitement sanguin, en particulier appareil de dialyse, destiné à exécuter un traitement sanguin, selon lequel du liquide est extrait du sang du patient par le biais d'au moins une membrane, **caractérisé en ce que** le dispositif de traitement sanguin comporte au moins un dispositif selon l'une des revendications 9 à 14 ou est formé par au moins un dispositif selon l'une des revendications 9 à 14.

Figur 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1444997 B1

- WO 2010112223 A1